# EUROPEAN PATENT APPLICATION

(11) **EP 1 769 769 A1**
(43) Date of publication of application: **04.04.2007**
(21) Application number: 05256033.1
(22) Date of filing: 28.09.2005
(51) Int. Cl.: A61B 19/00

(54) **Tracking surgical items**

(71) Applicant: DePuy Orthopädie GmbH, 85551 Heimstetten (DE)
(72) Inventor: The designation of the inventor has not yet been filed
(74) Representative: Alton, Andrew

(57) **Abstract**

A kit of parts, surgical item, method for adapting a surgical item and computer implemented method for tracking a marked surgical item are described. The kit of parts can include a surgical item, a marker assembly and at least one further marker separate to the marker assembly. The marker assembly comprises at least two markers and can be attached to the surgical item at a plurality of positions. The further marker can be mounted on the surgical item at a fixed position on the surgical item relative to a part of the surgical item to be tracked. When assembled, the position of said further marker relative to said marker assembly allows a tracking system to track the part of the surgical item with the marker assembly at any of the plurality of positions.

## Description

The present invention relates to markers for tracking surgical items, and in particular to methods and apparatus relating to marker arrangements allowing a surgical instrument to be more flexibly used and reliably tracked.

A star array, or other type of marker detectable by a tracking system, can be attached to a surgical item, such as a surgical tool, instrument, implant, *etc,* to allow the position of the item to be tracked by a tracking system. However, there can be problems for tracking systems relying on line of sight between the markers and the detector parts of the tracking system, as is the case for light based tracking systems, for example. Manipulation of the item, or a part of another item, a part of the operating room equipment or operating room personnel may impinge between the marker on the marked item and the tracking system detectors so that it is not possible for the tracking system to reliably track the item.

For example, it may be that the surgeon needs to manoeuver the item in a certain way in order to gain access to a surgical site such that the detectors can no longer 'see' the markers. Further, different operating room set ups (e.g. detector's position relative to patient, positions of assistants, surgeon or other tools) may require a variety of different marker positions on the surgical item in order to provide an optimal view of the item for the detectors. Changing the position of the marker on the item requires the tracking system to know the orientation of the marker array relative to the surgical item. This may be feasible for a limited number of possible marker positions. However, this can still lead to problems if the user enters the wrong position information into the computer system, or if the position of the marker is changed, accidentally or on purpose, during surgery.

The incorrect tracking of an instrument can have very serious consequences. For example in image guided surgical procedures, where accuracy is important, it could lead to a surgical procedure failing or harming the patient.

Further, changing the position of a marker and notifying the software introduces delays and further complexity to the surgical procedure.

Therefore, there is a need for a mechanism allowing the flexible use of marked surgical items while allowing reliable tracking thereof.

According to a first aspect of the invention, there is provided a kit of parts for providing a trackable surgical item. The kit of parts can include a surgical item, a marker assembly and at least one further marker. The marker assembly can comprise at least two markers and can be attachable to the surgical item at a plurality of positions. The at least one further marker can be separate to the marker assembly, which can be mountable on, or be provided on, the surgical item at a fixed position on the surgical item relative to the surgical item. When the kit of parts is assembled, the position of said further marker relative to said marker assembly allows a tracking system to track the surgical item with the marker assembly at any of the plurality of positions.

Hence, the marker assembly can be attached to the surgical item at various different positions, for ease of use of the item or as required by the field of view of the tracking system. The further marker has a fixed position on the surgical item relative to a part of the surgical item which it is desired to track. The marker assembly can be tracked and the relative position of the further marker and marker assembly can be determined in order to track the part of the surgical item, irrespective of where the marker assembly is attached to the item. Hence, the surgical item can be reliably tracked while having more flexible marker placement and without requiring the user to stop and reprogram the tracking system.

In a preferred embodiment, the marker assembly comprises at least three markers.

Preferably, the geometry of the further marker and the markers of the marker assembly does not correspond to a geometry that can be produce by a change in orientation of the instrument alone. The geometries can be different for all possible positions at which the marker assembly can be attached. Hence, it is possible to uniquely distinguish between the marker assembly being attached at a different position and the instrument simply having a different orientation. Herein, 'geometry' is generally used to refer to the spatial arrangement of the markers of the further marker and the marker assembly.

The kit of parts can further comprise a second further marker, separate to the marker assembly, which can be mounted on the surgical item at a fixed position on the surgical item relative to a part of the surgical item to be tracked. Providing a second further marker can be of benefit in helping to distinguish between different sides of an item.

The marker assembly can be adjustable so as to vary the spatial relationship between the three markers and the further marker. This can be of benefit in helping to distinguish between different sides of an item.

The marker assembly can be rotatable so that the three markers can have different angular positions. The three markers can have different angular positions when attached at different positions on the surgical item. A part of the marker assembly itself can be rotatable. For example, the array of markers can be rotatable about a support. The marker assembly can be indexed to identify different angular positions of the marker array. The marker assembly can be rotatable by being attached to the item after having been rotated through less than 360°.

The marker assembly can be tiltable so that the three markers can have different angular positions. The three markers can have different angular positions when attached at different positions on the surgical item. A part of the marker assembly itself can be tiltable. For example, the array of markers can be tiltable relative to a support part of the marker assembly. The marker assembly can be tiltable by being attachable to the item at different angles relative to a longitudinal axis of the item.

The fixed position can be on a longitudinal axis of the surgical item. This is particularly suitable for items being mirror symmetric about their longitudinal axis but not having rotational symmetry about their longitudinal axis.

The item does not need to be a surgical item and can be any item used during a surgical, medical or diagnostic procedure or treatment. For example, the item can be an instrument, tool or implant, including an orthopaedic implant, such as a prosthetic joint implant, for example a part of a prosthetic hip or knee.

According to a further aspect of the invention, there is provided a trackable surgical item. The trackable surgical item can comprise a surgical item, a marker assembly attached to the surgical item and a further marker attached to the surgical item. The marker assembly can comprise at least two markers. The marker assembly can be attachable to the surgical item at a plurality of positions. The further marker can be separate to the marker assembly be mounted on the surgical item at a fixed position relative to the surgical item. The position of said further marker relative to said marker assembly allows a tracking system to track the surgical item with the marker assembly at any of the plurality of positions.

The position of the further marker can be such that the geometry of the further marker and at least one of the markers of the marker array is not the same as the geometry of the markers of the marker array.

Preferred features of the surgical item can correspond to the preferred features of the kit of parts mentioned above.

According to a further aspect of the invention, there is provided a method for adapting a surgical item to be tracked by a tracking system. A first marker can be provided mounted on the surgical item at a fixed position relative to the surgical item. A marker assembly can be attached to the surgical item at a one of a plurality of different positions. The marker assembly can include at least two markers separate to the first marker. The position of said first marker relative to said marker assembly allows the tracking system to track the surgical item with the first marker assembly at any of the plurality of different positions.

According to a further aspect of the invention, there is provided a computer implemented method for tracking a surgical item using a tracking system. The surgical item can have a marker assembly attached thereto and a further marker attached thereto and separate to the marker assembly. The further marker can have a fixed positional relationship relative to the surgical item. The method can comprise determining the position of the marker assembly; determining the position of the further marker relative to the position of the marker assembly; and determining the position of the surgical item based on the position of the marker assembly, the position of the further marker relative to the marker assembly and the position of the further marker relative to the surgical item.

According to a further aspect of the invention, there is provided computer program code including instructions which can be carried out by a data processing device to provide the preceding method aspect of the invention. A computer program product comprising a computer readable medium bearing the computer program code aspect of the invention is also provided.

An embodiment of the invention will now be described, by way of example only, and with reference to the accompanying drawings, in which:
Figure 1A shows a schematic side view of a surgical item according to the invention bearing a marker arrangement according to the invention;
Figure 1B shows a schematic side view of a further surgical item according to the invention bearing a marker arrangement according to the invention;
Figure 1C shows a schematic side view of a yet further surgical item according to the invention bearing a marker arrangement according to the invention;
Figure 2A shows a perspective view of a further surgical item according to the invention bearing a marker arrangement according to the invention;
Figure 2B shows a perspective view of the surgical item of Fig. 2A from an opposite side;
Figure 3 shows a side view of a further surgical item according to the invention bearing a marker arrangement according to the invention;
Figure 4A shows a perspective view of a further surgical item according to the invention bearing a marker arrangement according to the invention;
Figure 4B shows a perspective view of the surgical item of Fig. 4A from an opposite side;
Figure 5 shows a process flow chart illustrating a computer implemented method according to the invention for tracking a surgical item as illustrated in the preceding Figures;
Figure 6 shows a schematic representation of images of the markers captured by a detector part of a tracking system; and
Figure 7 shows a schematic representation of the positional relationship between the markers and instrument tip in a reference frame of the tracking system.

Similar items in different Figures share common reference numerals unless indicated otherwise.

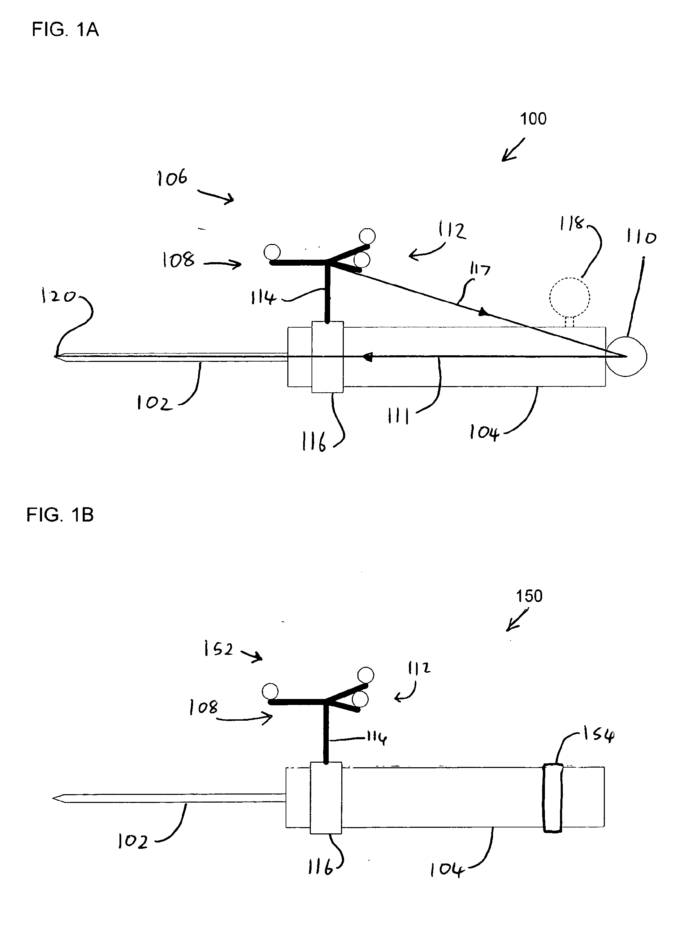

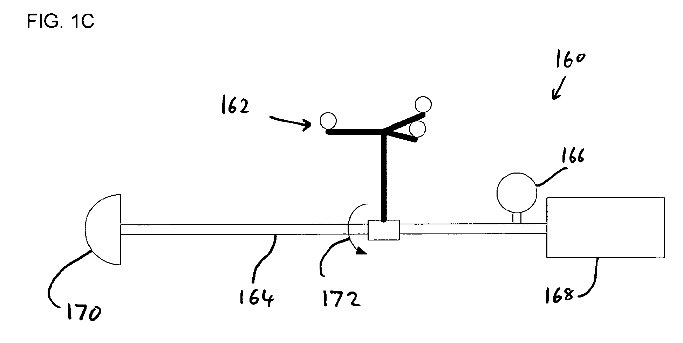

Figure 1A shows a schematic side view of a surgical item 100 bearing a marker arrangement. The surgical item 100 is an instrument, and in particular, a pointer or probe which is rotationally symmetric about its longitudinal axis. The instrument includes a pointer part 102 and a right circular cylindrical symmetric handle part 104. The instrument bears a marker arrangement 106 comprising a marker assembly 108, also referred to as a marker or star array, and a further marker 110.

Marker assembly 108 comprises an array of three reflective spheres 112 on a support 114 including a clamp 116 by which the marker assembly can be attached to the instrument. The array can be attached to the instrument at various positions along the longitudinal axis of the handle and also at different angles about the longitudinal axis of the handle.

It is possible to track some instruments, e.g. a pointer, using a marker assembly having only two markers. Therefore in some embodiments of the invention, a marker assembly including only two reflective spheres on a support can be used. Further, in other embodiments, marker assemblies using more than three markers can be used.

Further marker 110 is also in the form of a reflective sphere and is attached at a proximal end of the handle with its centre lying on the longitudinal axis of the instrument. Also shown in Figure 1A, in dashed lines, is an alternate position for an alternate further marker 118. In this alternate embodiment, the marker is also in the form of a reflective sphere which is mounted on a short stub on the handle of the instrument. The position of the further marker 110, 118 is fixed with respect to the instrument.

The instrument shown in Figures 1A and 1B can be used with tracking systems that detect reflected light and can be considered passive markers. Other embodiments can include active markers in which the markers themselves generate and emit light rather than reflecting incident light. In other embodiments, acoustic tracking can be used and the markers can provide an active or passive source of an acoustic signal.

As the further marker 110 has a fixed position on the instrument, the position of the further marker 110 relative to the tracked part of the instrument, e.g. tip 120, is known and can be stored by the tracking system. For example, the position of fixed further marker 110 relative to tip 120 can be represented by a position vector 111 which defines the distance and direction from the centre of marker sphere 110 to the tip 120 of the instrument. In use, a surgeon may decide that they want to hold the probe closer to the tip end and so may release clamp 116 and move the star array 112 closer to the proximal end of the handle. In a conventional marked instrument, this system would no longer know the position of the marker array relative to the instrument and so tracking of the instrument tip would no longer be reliable.

However, in the present invention, as there is a fixed positional relationship between the further marker and the instrument, and the further marker and marker array can be tracked by the tracking system, the tracking system can use the detected position of the further marker and the detected position of the marker array to reliably track the instrument position in space. In particular, the tracking system determines the position of the further marker 110 relative to the marker array 112, as illustrated by vector 117 in Figure 1A. The tracking system can then determine the position and orientation in space of the tip 120 of the instrument as the position of the tip relative to the further marker is known and given by vector 111.

Figure 1A also illustrates an alternate embodiment in which the further marker 118 is mounted on the handle of the instrument, rather than on its end. In this case, vectors 117 and 111 correspond to the relative position of the centre of the array and the position of the centre of further marker 118, and the position of the centre of further marker 118 and position of the tip 120.

Figure 1B shows an alternate embodiment 150 of an instrument similar to that shown in Figure 1A but bearing a different marker arrangement 152. In this embodiment, the further marker 154 is in the form of narrow strip of reflective material extending around the longitudinal axis of the instrument. The tracking system knows the fixed positional relationship between the tip of the instrument and the reflective material, and can then determine the vector between the marker array and the reflective strip. Hence, the marker array can be mounted at any position along the longitudinal axis of the instrument as convenient for tracking.

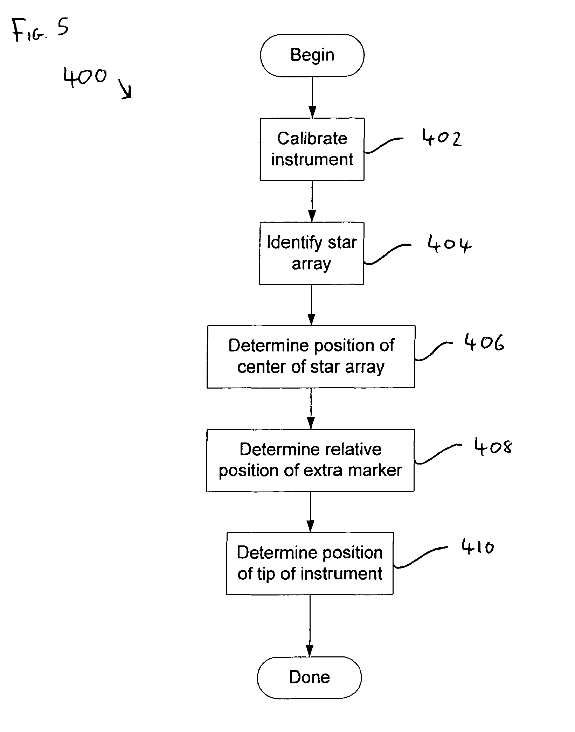

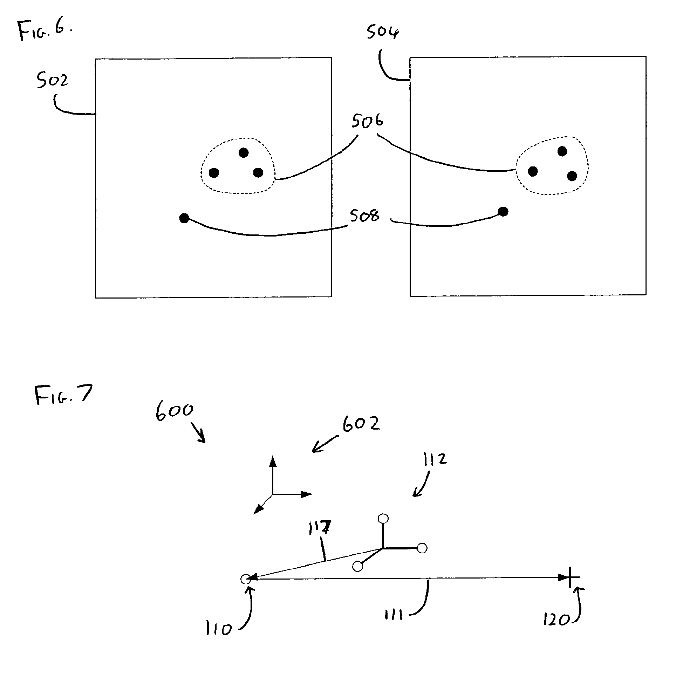

How the tracking system tracks the marked instrument will now be described with reference to Figures 5, 6 and 7 in particular. Figure 5 shows a high level process flow chart illustrating a computer implemented method 400 for tracking a marked item. Figure 6 shows a schematic representation of the two images 502,504 captured by two detectors of the tracking system and Figure 7 shows a schematic representation 600 of the positional relationship between the markers in a tracking space, reference frame or coordinate system of the tracking system.

If an instrument is not already calibrated, i.e. the tracking system does not know the positional relationship between the fixed marker and the working part of the instrument, e.g. tip 120, then at step 402, the instrument can be calibrated. A calibration jig is provided and includes a marker array which can be tracked by the tracking system so that the tracking system can determine the position of the calibration jig in the reference frame of the tracking system. The calibration jig includes a calibration formation, such as a dimple, for receiving the working part, such as tip 120, of the instrument to be calibrated. The tracking system knows the position of the calibration formation relative to the calibration jig marker array and so can determine the position of the calibration formation in its reference frame.

The instrument to be calibrated is inserted in the jig with its working part engaging the calibration formation and the fixed further marker is presented to the detectors of the tracking system. The tracking system then determines the position in its reference frame of the fixed further marker. The working part of the instrument is engaged with the calibration formation and so the position of the working part in the tracking system's reference frame is the same as the position of the calibration formations' position. Hence the tracking system can determine the position of the working part of the instrument, tip 120, relative to the fixed marker 110 and stores this calibration information for the instrument.

As mentioned above, if the instrument is pre-calibrated then step 402 can be skipped and so is optional.

The marker array 112 is attached to the instrument at any position as preferred by the user and the tracking system can start tracking the instrument along with any other marked items in its field of view. The tracking system includes a source of infrared radiation and two spaced apart infrared detectors or cameras which capture infrared images in synchrony with the frequency the infrared source. Figure 6 shows a schematic representation of the parts of the images 502, 504 captured by the infrared detectors of the marked instrument. As can be seen each image includes four 'dots' each corresponding to a respective one of the reflective spheres. As the detectors are spaced apart, each detector has a slightly different view of the instrument and so stereoscopic data is provided from which positional information can be determined by the tracking system in a manner known to those of ordinary skill in the art.

The tracking system has access to a database which stores geometric information about each of the marker arrays that the tracking system can recognise and track, including the marker array attached to the instrument. At step 404, the tracking system determines which group of three dots 506 corresponds to the geometry of a one of the marker arrays. As the marker arrays each have a unique geometry, only a one of them can correspond to three of the dots as seen by both detectors. Hence, at step 404 a one of the marker arrays is identified and as part of the process of fitting the marker array to the images, the position and orientation in space of the marker array is also determined. Then, at step 406, using the geometry of the marker array, and the position and orientation of the marker array in space, the centre of the marker array, or some other reference point of the marker array, is determined. Then, the position of the further marker, corresponding to the remaining dot 508 in the images 502, 504, is determined. Then, at step 406 the position of the further marker relative to the centre of the marker array is determined.

The position of the tip of the instrument in the reference frame of the tracking system can then be determined at step 410, as illustrated in Figure 7. Figure 7 shows a schematic representation 600 of the reference frame 602 of the tracking system, including the position of the centre of the marker array 112, the position of the further marker 110, and the position of the tip 120. The position and orientation of the marker array is continuously determined by the tracking system. The position of the centre of the marker array is known at any time, the relative position of the further marker and centre of the marker array has been determined and corresponds to vector 117, the position of the tip relative to the further marker is stored and known by the tracking system and corresponds to vector 111. Hence, the position of the tip in the reference frame of the tracking system at any point in time corresponds to the position of the centre of the marker array plus vector 117 plus vector 111.

If reference array 112 is moved at any time during use, for example, to make it easier to hold or use the instrument, the tracking system simply re-determines the positions of the centre of the marker array relative to further marker 110, that is vector 117, in the reference frame of the tracking system, and hence the position of the tip can continue to be accurately tracked.

Figure 1C shows a schematic side view of an acetabular cup inserter instrument 160 which includes a marker array 162 attached to a body part 164, a further marker 166 attached to the body part adjacent a handle part 168 and an acetabular cup trial or implant component 170 attached to a distal end of the instrument. As mentioned above, in some embodiments a two marker linear marker assembly can be fixed to the instrument.

As illustrated in Figure 1C by arrow 172 marker assembly 162 can be rotated around the longitudinal axis of the instrument and can also be translated along the longitudinal axis and then clamped in place, so as to optimally position the marker array for tracking. After calibration of the instrument, so that the vector between the marker array and the further marker is known by the tracking system, it is possible to navigate the cup 170 position as there is a fixed vector between the cup on the instrument and the position of the fixed further marker 166.

For rotationally symmetric tools as illustrated in Figures 1A to 1C the tracking system only needs to store data representing the vector, e.g. 111, between the fixed further marker and the working point of the instrument. The tracking system can then derive the vector between the centre of the marker array and the further marker. For such instruments, it is not necessary to differentiate between a rotation of the marker array relative to the instrument and a rotation of the instrument itself. The marker array can be rotated relative to the axis of rotational symmetry of the tool and translated along that axis so as to located the marker array at any possible position.

A similar approach can be used for instruments which are not rotationally symmetric about their longitudinal axes.

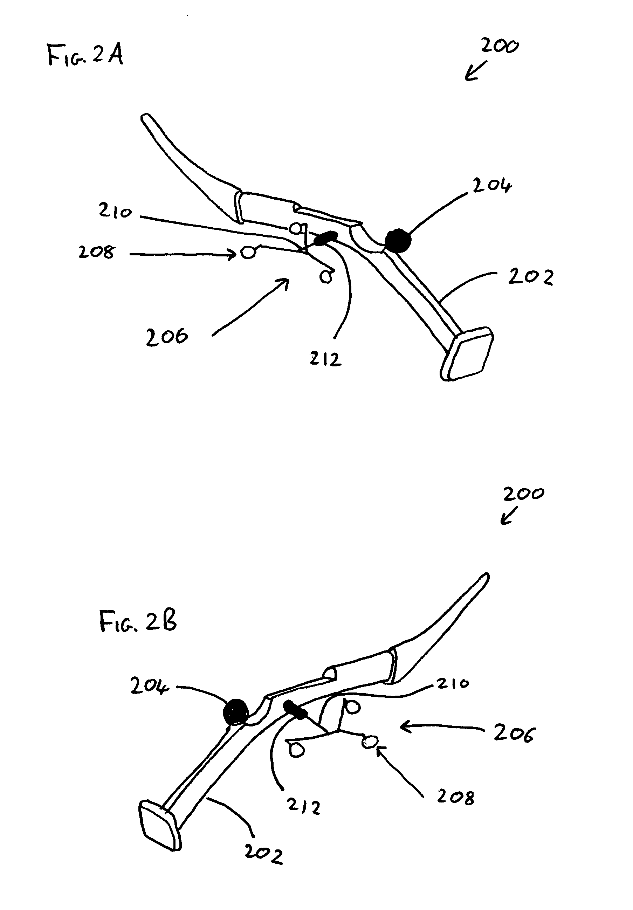

Figures 2A and 2B respectively show perspective views of a further instrument 202 according to the present invention from different sides. The instrument 202 is a broach handle with a femoral broach attached. The instrument bears a marker arrangement comprising a first marker 204 fixed to a handle part of the instrument. Marker 204 is in the form of a sphere with a reflective coating. The marker arrangement also includes a marker assembly 206 comprising a marker array 208 on a support 210 and a connector 212 by which the marker assembly can be connected to a first side of the instrument (as shown in Figure 2A) or a second opposite side of the instrument (as shown in Figure 2B). Hence the marker array assembly can be attached to the instrument at a one of a plurality of positions and the marker 204 is attached to the instrument at a fixed position. There is therefore a well defined positional relationship between the fixed marker 204 and the marker array when attached to the instrument at any of its different positions.

In use, the surgeon attaches the marker array to the side of the instrument which, in use, will be in the field of view of the detectors of the tracking system. In the absence of the further marker 204, a tracking system would be able to identify the position and orientation of the marker array but would not be able to determine on which side of the instrument the marker array was attached. However, the pattern of dots generated by the marker array and further marker with the marker array on a first side is different to the pattern of dots generated by the fixed marker 204 and marker array with the marker array on the other side and therefore the tracking system can distinguish between the two cases and uniquely determine the orientation and position of the instrument.

In some cases, the tracking software may not be able to distinguish between the array being placed in a different position on the instrument and a particular attitude, or orientation, of the instrument in space. This is not the case for the above described embodiment as the array can only be mounted on either side of the instrument and so the tracking system will only be able to recognise the array together with the additional marker if they are on the side facing the camera. Approaches for addressing the special case problem will be described below.

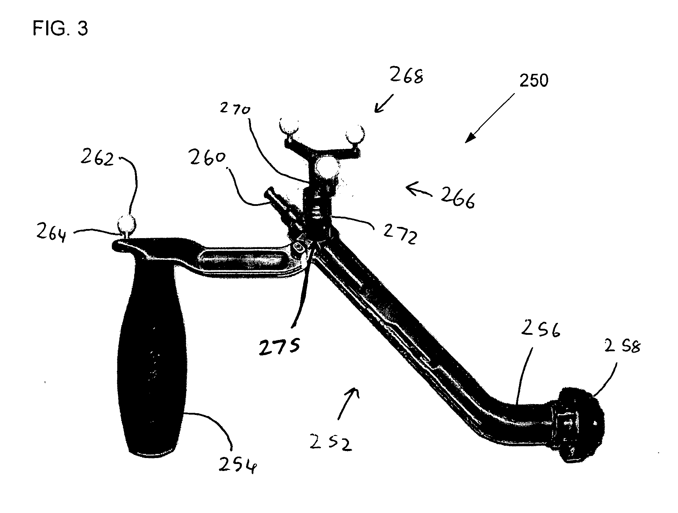

Figure 3 shows a side view of an acetabular reamer instrument bearing a marker arrangement 250 according to the present invention. The reamer instrument 252 has a handle part 254 and a reamer part 256 with a reamer head 258 at a distal end and a power connector 260 at a proximal end. A marker 262 in the form of a reflective sphere is attached to the reamer by a stub 264 at a fixed position adjacent the handle 254. The fixed marker 262 is not positioned on either side of the instrument but rather sits on the lateral plane of the instrument. A marker assembly 266 is also provided and comprises a marker array 268 including three spherical reflectors mounted on a support 270 and a connector 272. The marker array can be adjusted and locked in a number of positions around a shoulder part of the reamer instrument. For example the number of positions can be from three to five. As illustrated in Figure 3, the marker assembly is at a right hand side position of the instrument.

It is possible that the spatial distribution of the markers detected by the tracking system will not provide a unique system to distinguish between the attitude of the instrument in space and the reference array attached at different positions. In this case, the following considerations should be borne in mind to mitigate this problem. Several of the following options, or combinations of them, can be used to provide a unique solution.

In general, the geometry of the marker arrays used on the instruments is stored in a database accessible by the software. In general, 'geometry' is used herein to refer to the spatial arrangement of the markers of the marker array or of the further marker and the markers of the marker array. The navigation system detects the three markers on any marker array and from them determines the centre of the of the array, which has a known geometry, and the orientation of the marker array.

In contrast to the rotationally symmetric instrument case, it is important to distinguish between a different position of the marker array on the instrument and just a different orientation of the instrument relative to the cameras of the tracking system.

The vectors from the marker array to the part of the instrument being tracked or navigated (e.g. the tool tip) for each of a finite number of different positions of the marker array are stored in the database. The vector from the further marker to the tracked part of the instrument is also stored in the database. The vector or vectors from the further marker to the centre of the marker array at the plurality of marker array positions are also stored in the database.

The spatial distribution of the dots created by the fixed marker and the marker array in the captured images is processed by the tracking system to automatically determine in which of the possible positions the marker array is mounted. There are only a limited number of positions at which the marker array can be mounted. The vector between the marker array and the further marker is determined from the captured images. The vector so determined is then compared with the plurality of vectors between the marker array at its different possible positions and the further marker stored in the database. The position of the marker array is then determined to be the position whose corresponding stored vector most closely matches the vector determined from the images. Hence, the particular position at which the marker array is attached has been determined.

The instrument can therefore be navigated using the tracked position of the marker array and either: the vector from the marker array to the tool tip for the now known position of the marker array on the instrument; or the vector from the now known position of the marker array to the further marker and the vector from the further marker to the tool tip. In other embodiments the vectors between the marker array at its plurality of positions and the tool tip need not be stored in the database. However, if they are available, then the appropriate vector can be used to check if it "closes the loop" when added to the other two vectors to ensure that the instrument is still calibrated, ie the tool tip still has its original spatial position in relation to the further marker and/or the marker array positions.

A first consideration, in order to help prevent the further marker from being identified as being a marker of the marker array, is that the further marker should be positioned so that the additional marker and any two of the markers of the marker array do not have the same geometry as the three markers of the marker array. This can be achieved, for example, by ensuring that the further marker is further from the centre of the triangle formed by the markers of the marker array than any of the markers of the marker array.

A further consideration is to arrange the geometry of the further marker and marker array so as to help distinguish between the marker array being in a different position on the instrument and the instrument being in a different position and/or orientation. It may not be possible to distinguish between these two scenarios if, for example, the instrument is rotated around an axis passing through the further marker and through a point which corresponds to the axis perpendicular to the plane of the marker array and passing through the centre of the plane of the marker array. For example, in Figure 3, this would be the axis passing through the centre of further marker 262 and where the base 272 of the marker array 272 joins the instrument. If the marker array 268 can be positioned at numerous different positions around that axis, without other wise changing the geometry, then it would not be possible to distinguish between the marker array being in different positions relative to the instrument and rotation of the instrument around that axis.

However, this can be overcome by ensuring that the symmetry between the different positions of the marker array and rotation of the instrument is broken. For example, if the marker array is moved around the axis, but the markers of the array are also rotated about the axis perpendicular to the plane of the array, then there is no longer symmetry between the geometry of the further marker and marker array in the first and second positions and rotation of the instrument about the axis. Hence, in scenarios in which changing the position of the marker array relative to the instrument can coincide with a simple change in orientation of the instrument, the marker array should be altered so as to change the geometry of the further marker and marker array so that the change in position of the marker array can be uniquely identified.

The above scenario can be avoided by simply ensuring that the different positions of the marker array on the instrument are not symmetric with a change in orientation of the instrument, for example by providing further possible positions of the marker array which do not map onto a change in orientation of the instrument, for example attachment point 275 in Figure 3.

This is also the case in Figure 2A and Figure 2B. In Figure 2A, the long arm of the marker array is pointing away from the further marker 204 and in Figure 2B the long arm if pointing toward the further marker 204. Hence the geometry of the markers is different with the marker array on different sides of the instrument and is not symmetric with a rotation around the axis of the instrument. Hence, it is possible reliably to distinguish between the marker array being in different positions and a simply rotation of the instrument.

Another option for addressing this problem include the use of a second marker fixed to the instrument. For example, marker 262 would be attached on one side of the handle and a similar marker would be attached on another side of the handle so as to enable the tracking system to uniquely determine which position the marker array is at, so as to uniquely determine the position and orientation in space of the instrument. A third, or further markers, having fixed positions on the instrument could also be used.

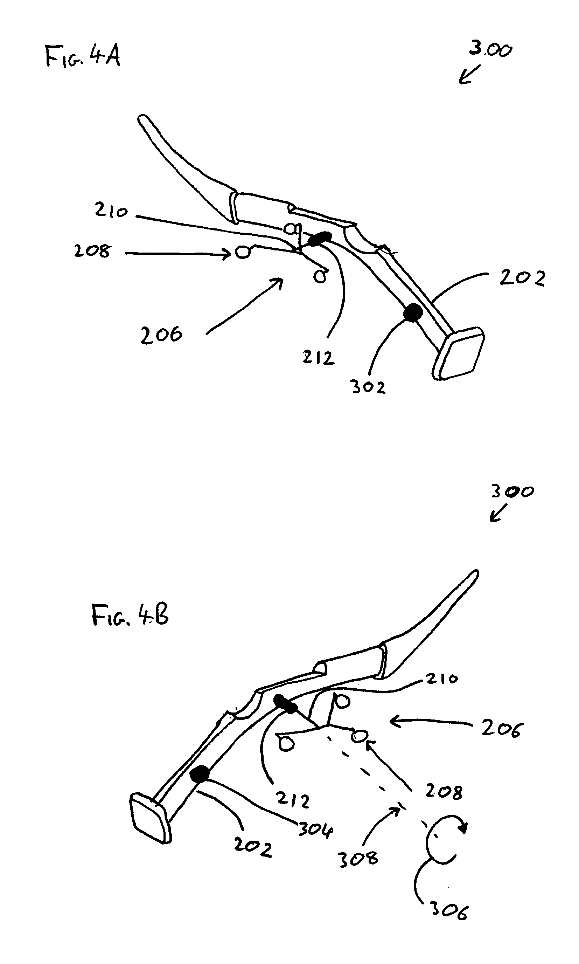

An example of this approach is illustrated in Figures 4A and 4B which show an instrument 300 similar to instrument 200 shown in Figures 2A and 2B. However, in this alternate embodiment, a first marker 302 is fixed to a first side of the instrument and a second marker 304 is fixed to the second opposite side of the instrument. Hence in this embodiment, two fixed markers are used so as to help distinguish between the two positions of the marker assembly as the array of the marker assembly presents a different pattern to the detector depending on which side of the instrument it is attached to.

A further option is to use a different distance between the fixed marker and the centre of the marker array. For example the first fixed marker 302 and the second fixed marker 304 can be attached at different positions on the handle of the instrument so that the separation between the fixed marker 302, 304 and the centre of the array is different with the array positioned on either side of the instrument. Alternatively, the fixed markers can have the same position on the handle of the instrument and the marker array can be attached at different positions on the first and second sides of the instrument so as to provide different separations between the centre of the marker array and the fixed marker.

A further approach involves rotating the marker array, as indicated by arrow 306 about an axis 308 transverse to the plane of the marker array so that the marker array presents a different pattern depending on which of the plurality of the positions on the instrument it is attached at. For example if there are N different positions at which the marker array can be attached to the instrument, then there should be N different degrees of rotation possible for the marker array. For example, if there are four different positions, then the marker array can be rotated through 0, 90, 180 and 270 degrees for respective positions. In one embodiment this is achieved by pivotally mounting the marker array so that it can rotate about its support and providing indexation points every 360° divided by N to provide N different rotational positions for the marker array.

In another approach, the instrument can be provided with a coupling to which the marker array is attached and in which the coupling is rotatable and has a shape with a sense of direction. For example, the coupling can have a generally D shape. This constrains a cooperating coupling on the marker array so that the marker array can only be attached to the coupling on the instrument in a certain direction. The orientation of the marker array relative to the instrument can then be changed by rotating the coupling part on the instrument. For example, the instrument coupling can be a D shaped female formation and the marker array coupling can be a D shaped make formation. In another embodiment, the instrument coupling can be fixed relative to the instrument so as to prevent rotation so that the array can only be attached with a certain orientation relative to the instrument. Multiple fixed couplings can be provided on the instrument at different positions and/or orientations.

A further option would be to add a further tilt degree of freedom to the marker array assembly. Hence when attached in a first position the marker assembly can adopt a first attitude and when attached at a different position, the marker array can be tilted so that its plane is no longer transverse to the longitudinal axis of the support and adopts a different attitude.

Generally, embodiments of the present invention also employ various processes involving data stored in or transferred through one or more computer systems. Embodiments of the present invention also relate to an apparatus for performing these operations. This apparatus may be specially constructed for the required purposes, or it may be a general-purpose computer selectively activated or reconfigured by a computer program and/or data structure stored in the computer. The processes presented herein are not inherently related to any particular computer or other apparatus. In particular, various general-purpose machines may be used with programs written in accordance with the teachings herein, or it may be more convenient to construct a more specialized apparatus to perform the required method steps. A particular structure for a variety of these machines will appear from the description given above.

In addition, embodiments of the present invention relate to computer readable media or computer program products that include program instructions and/or data (including data structures) for performing various computer-implemented operations. Examples of computer-readable media include, but are not limited to, magnetic media such as hard disks, floppy disks, and magnetic tape; optical media such as CD-ROM disks; magnetooptical media; semiconductor memory devices, and hardware devices that are specially configured to store and perform program instructions, such as read-only memory devices (ROM) and random access memory (RAM). The data and program instructions of this invention may also be embodied on a carrier wave or other transport medium. Examples of program instructions include both machine code, such as produced by a compiler, and files containing higher level code that may be executed by the computer using an interpreter.

## Claims

1. A kit of parts for providing a trackable surgical item, the kit of parts including:
a surgical item;
a marker assembly comprising at least two markers and which can be attached to the surgical item at a plurality of positions; and
at least one further marker, separate to the marker assembly, which can be mounted on the surgical item at a fixed position on the surgical item relative to a part of the surgical item to be tracked, wherein, when assembled, the position of said further marker relative to said marker assembly allows a tracking system to track the part of the surgical item with the marker assembly at any of the plurality of position.

2. The kit of parts as claimed in claim 1, wherein the geometry of the further marker and the markers of the marker assembly when attached at any of the plurality of positions does not correspond to a geometry that can be produce by a change in orientation of the instrument alone.

3. The kit of parts as claimed in claim 1 or 2, and further comprising:
a second further marker, separate to the marker assembly, which can be mounted on the surgical item at a fixed position on the surgical item relative to a part of the surgical item to be tracked.

4. The kit of parts as claimed in any of claims 1 to 3, wherein the marker assembly is rotatable so that the markers can have different angular positions when attached at different positions on the surgical item.

5. The kit of parts as claimed in any of claims 1 to 4, wherein the marker assembly is tiltable so that the markers can have different angular positions when attached at different positions on the surgical item.

6. The kit of parts as claimed in any preceding claim, wherein the fixed position is on a longitudinal axis of the surgical item.

7. The kit of parts as claimed in any preceding claim, wherein the surgical item is a surgical instrument.

8. The kit of parts as claimed in claim 7, wherein the surgical instrument is not rotationally symmetric about its longitudinal axis.

9. A trackable surgical item, the comprising:
a surgical item;
a marker assembly attached to the surgical item and comprising at least two markers and which can be attached to the surgical item at a plurality of positions; and
at least one further marker, separate to the marker assembly, and mounted on the surgical item at a fixed position on the surgical item relative to a part of the surgical item to be tracked, wherein the position of said further marker relative to said marker assembly allows a tracking system to track the part of the surgical item with the marker assembly at any of the plurality of positions.

10. A trackable surgical item as claimed in claim 9, in which the position of the further marker is such that the geometry of the further marker and at least one of the markers of the marker array is not the same as the geometry of the markers of the marker array.

11. A trackable surgical item as claimed in claim 9 or 10, wherein the geometry of the further marker and the markers of the marker assembly when attached at any of the plurality of positions does not correspond to a geometry that can be produce by a change in orientation of the instrument alone.

12. A method for adapting a surgical item to be tracked, comprising:
providing a first marker mounted on the surgical item at a fixed position on the surgical item relative to a part of the surgical item to be tracked by the tracking system; and
attaching a marker assembly to the surgical item at a one of a plurality of different positions on the surgical item, the marker assembly including at least two markers separate to the first marker, wherein the position of said first marker relative to said marker assembly allows the tracking system to track the surgical item with the first marker assembly at any of the plurality of different positions.

13. A computer implemented method for tracking a surgical item using a tracking system, the surgical item having a marker assembly including at least two markers attached thereto and a further marker attached thereto and separate to the marker assembly and having a fixed positional relationship relative to a part of the surgical item being tracked, the method comprising:
determining the position of the marker assembly in a reference frame of the tracking system;
determining the position of the further marker in the reference frame of the tracking system relative to the position of the marker assembly; and
determining the position of the part of the surgical item in the reference frame of the tracking system based on the position of the marker assembly, the position of the further marker relative to the marker assembly and the position of the further marker relative to the part of the surgical item.

14. Computer program code including instructions which can be carried out by a data processing device to provide the method of claim 13.

15. A computer program product comprising a computer readable medium bearing computer program code as claimed in claim 14.
